# NEW EUROPEAN PATENT SPECIFICATION

(11) **EP 0 109 178 B2**
(45) Date of publication and mention of the opposition decision: **26.01.2000**
(45) Mention of the grant of the patent: 27.05.1992
(21) Application number: 83306120.3
(22) Date of filing: 10.10.1983
(51) Int. Cl.: A61N 1/05, A61M 25/01

(54) **Flexible tip cardiac pacing catheter**
Katheter für Herzschrittmacher mit biegsamer Spitze
Cathéter d'entraîneur cardiaque avec pointe flexible

(30) Priority: 14.10.1982 US 434318
(43) Date of publication of application: 23.05.1984
(60) Divisional application: 87114978.7
(73) Proprietor: BAXTER INTERNATIONAL INC., Deerfield, IL 60015 (US)
(72) Inventor: Swendson, David Lyman, Garden Grove Ca 92645 (US); Elson, Edward Earl, Anaheim California 92806 (US); Lieber, Clement Eugene, Yorba Linda CA 92686 (US); Rold, Michael D., Costa Mesa CA 92627 (US)
(74) Representative: Day, Jeremy John

(56) References cited:
- EP-A- 0 057 448
- WO-A-81/03733
- WO-A-83/04181
- DE-A- 2 605 590
- DE-A- 2 822 829
- DE-A- 3 026 936
- US-A- 3 416 533
- US-A- 3 596 662
- US-A- 3 788 329
- US-A- 3 804 098
- US-A- 3 995 623
- US-A- 4 161 952
- BIOMEDIZINISCHE TECHNIK, vol.24, no. 1/2, 1979, pages 16-27
- ZEITSCHRIFT FÜR KREISLAUFFORSCHUNG, vol. 61, no. 10, 1972, pages 887-891

## Description

### BACKGROUND OF THE INVENTION

A pacing catheter and a pulse generator are used to electrically stimulate or pace the heart. To accomplish this, the catheter is inserted through a vein into the heart. Typically, the catheter is inserted into the right ventricle. The catheter may be either unipolar, i.e., have one electrode, or bipolar, i.e., have two electrodes. In either event, the distal electrode of the catheter must be brought into contact with the heart wall in order that pulses of electrical energy can be transmitted from the pulse generator through the catheter to the heart. Pacing of the heart in this fashion is often temporary and may be required, for example, in surgery following a myocardial infarction.

One problem with pacing catheters is that the insertion of the catheter through the heart and into engagement with the heart wall creates a risk of penetration of the heart wall by the catheter. The risk of penetration cannot be avoided by making the catheter uniformly flimsy because the catheter must have enough stiffness to be inserted into the heart. In addition, the catheter must have some resilience so that it can maintain the electrodes in substantially continuous contact with the heart wall in the presence of factors such as the beating of the heart and patient movement, which tend to interrupt engagement between the electrodes and the heart wall.

It is known to provide a flexible tail on a heart stimulation catheter and to space the electrodes proximally from the tail as shown in U.S. -A- 3,664,347. In this construction, the tail must be in an artery of the lungs and neither of the electrodes is at or near the distal end of the catheter.

Other constructions of catheters with flexible electrodes are disclosed in U.S.-A-3416533 and U.S.-A-3788329.

### SUMMARY OF THE INVENTION

This invention overcomes the pacing catheter insertion problem described above by providing a catheter with a flexible, resilient distal end portion and a flexible electrode. The distal end portion of the catheter has insufficient column strength to perforate the heart wall when the distal end portion is axially pushed against the heart wall. When the distal end portion contacts the heart wall, it bends or deflects and guides the distal end along the heart wall rather than through it. Also, in this bent-over condition, a larger surface area of the distal end portion engages the heart wall thereby decreasing unit loading and making heart wall perforation much less likely to occur.

With this invention, the resilient, flexible characteristic of the distal end portion extends all the way to the distal end of the catheter.

The catheter of this invention is as defined in claim 1 hereunder.

In a preferred implementation, the conductor includes an elongated wire with a distal section and a transition section of the conductor being wound into a coil with the coil turns of the transition section being spaced greater axially than the spacing between the coil turns of the distal section. This provides the transition section with greater rigidity and less flexibility than the distal section. Alternatively, or in addition thereto, to enhance flexibility of the wire at distal locations, the cross-sectional area of the wire can be progressively reduced as the wire extends distally. The first section of the wire is straight, i.e. uncoiled.

The transition section is, therefore, a stiffness transition zone between the relatively stiff first section and the relatively flexible distal section. This transition zone prevents high stress points between the relatively stiff and relatively flexible sections. Without this transition section, permanent deformation is more likely to occur and fatigue strength is reduced.

The features of this invention are applicable to a unipolar or bipolar catheter. In one preferred form of bipolar catheter, the body includes a conductive wire having a plurality of coils with a region of the coils being exposed to define a second electrode. Preferably this wire is rectangular in cross section to increase the stiffness of the body and reduce its electrical resistance.

If desired, the interior of the coils can be filled with an elastomeric material to aid in controlling the flexibility of the distal end portion of the catheter. Also, the elastomeric material can extend beyond the distal tip of the coils to provide a soft distal end to the catheter. Filling of the interior of the coils also prevents the ingrowth of tissue into the coils. Finally, in a bipolar catheter, the elastomer can provide insulation between the two conductors, and by axially spacing coils of the spring forming the second electrode, the elastomer can be provided between the spaced coils to tend to lock the structure together.

The flexible tip catheter of this invention can be inserted without any guiding implement through a vein into the heart. Alternatively, the catheter can be advanced through the lumen of an indwelling right heart monitoring catheter and exit at a side port in either the ventricle or atrium to perform its functions.

The pacing catheter of this invention can be used separately or in combination with a guiding catheter. Also, the guiding catheter can be used with other pacing catheters, and the pacing catheter of this invention can be used with other guiding catheters.

The invention, together with additional features and advantages thereof, may best be understood by reference to the following description taken in connection with the accompanying illustrative drawing.

### BRIEF DESCRIPTION OF THE DRAWING

Fig. 1 is a side elevational view partially in section of a bipolar catheter constructed in accordance with the teachings of this invention coupled to a pulse generator.
Figs. 2a and 2b are fragmentary side elevational views showing two different forms of wire which can be used for the conductor.
Figs. 3a and 3b are side elevational views of two forms of bipolar flexible tip catheters of this invention, respectively. The elastomeric material within the coils is not shown in Figs. 3a and 3b.
Fig. 4 is a front elevational view of a unipolar catheter constructed in accordance with the teachings of this invention.
Fig. 5 is a sectional view through a human heart showing one way in which the catheter of this invention can be used.

### DESCRIPTION OF THE PREFERRED EMBODIMENTS

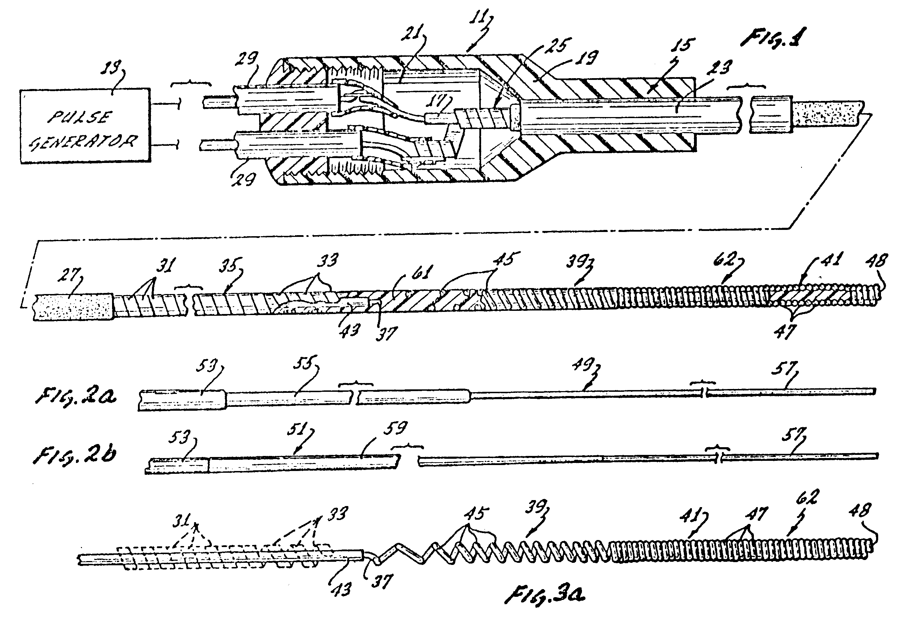

Fig. 1 shows a bipolar catheter 11 electrically coupled to a pulse generator 13. Generally, the catheter 11 comprises a body 15 and an elongated inner conductor 17.

The body 15 includes a backshell 19 having an interior chamber 21, a tube 23 received within one end of the backshell 19, an outer conductor 25 and a flexible outer cover 27 of a suitable material, such as polytetrafluoroethylene. The conductors 17 and 25 are suitably coupled within the chamber 21 to leads 29 leading to the pulse generator 13. The chamber 21 may contain a suitable potting compound (not shown) if desired.

In the embodiment illustrated, the outer conductor 25 is in the form of a flat wire, i.e., a wire of nearly rectangular cross section, wound into a series of contiguous coil turns 31 which extend from the chamber 21 to a location distally of the cover 27 and axially spaced coil turns 33 located distally of the contiguous coil turns 31. The cover 27 terminates proximally of the distal end of the conductor 25, and the exposed portion of this conductor forms an electrode 35.

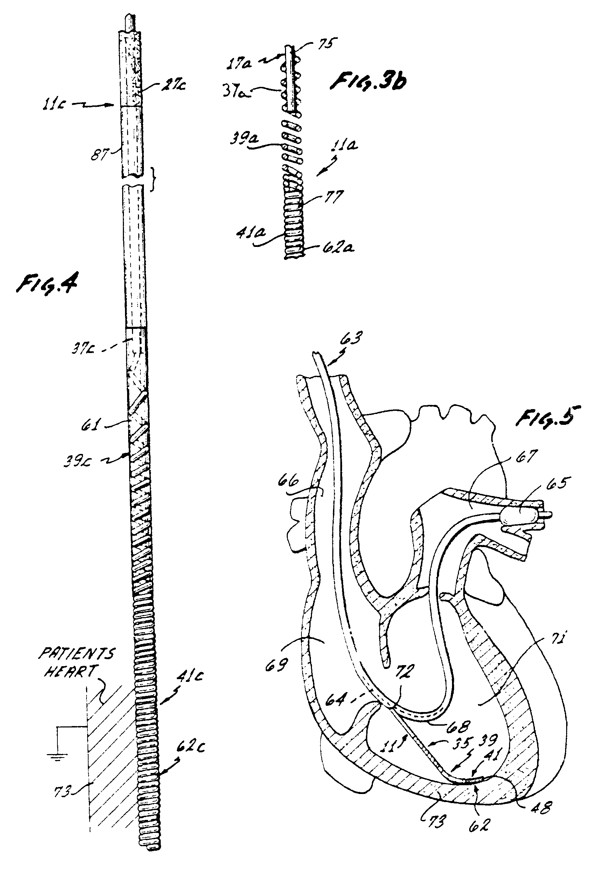

The inner conductor 17 is also in the form of an elongated wire having a first section 37 extending from the chamber 21 axially through the body 15, a transition section 39 and a distal section 41 with the portion of the inner conductor 17 which is outside of said body being the distal end portion of the inner conductor. As best shown in Fig. 3a, the first section 37 is straight and is covered by insulation 43. The transition section 39 comprises a plurality of coil turns 45 which are spaced apart axially, with the axial spacing between adjacent coil turns progressively reducing as the transition section extends distally. This progressive reduction in spacing is preferred but not essential. The distal section 41 comprises a plurality of contiguous coil turns 47 and terminates at a distal end 48 of the catheter 11. These coil turns could be tightly or loosely wound to further control flexibility.

The inner conductor 17 may be formed, for example, of a wire 49 (Fig. 2a) or of a wire 51 (Fig. 2b). The wire 49 has a cylindrical section 53 of relatively large diameter which can be used to form the first section 37 and cylindrical sections 55 and 57 which can be used to form the transition section 39 and the distal section 41, respectively. Each of the sections 55 and 57 is of lesser diameter than the cylindrical section immediately proximally thereof.

The wire 51 also has a cylindrical section 53 from which the first section 37 can be formed. However, in lieu of the cylindrical section 55, the wire 53 has a conical section 59 which is of progressively reducing diameter as it extends distally and from which the transition section 39 can be constructed. The wire 53 also has a cylindrical section 57 from which the distal section 41 can be constructed. The wires 49 and 51 can be constructed of various suitable materials, such as stainless steel. The wires 49 and 51 can be formed of multiple sections which are suitably joined together as by soldering or welding but, preferably, each of these wires is integral. In this latter event, the wires 49 and 51 can be tapered by electropolishing or centerless grinding.

With the construction described above, the first section 37 is less flexible than the transition section 39 and the transition section 39 is less flexible than the distal section 41. Also, the first section 37 is reinforced by the body 15. Moreover, the flexibility of the transition section 39 increases as the transition section extends distally. The relative stiffness of the first section 37 is obtained by leaving the first section 37 uncoiled and constructing it of larger diameter wire. The transition section 39 is more flexible than the first section 37 because it is coiled and constructed of smaller diameter wire. The flexibility of the transition section 39 increases because the axial spacing between the coil turns 45 decreases as the transition section extends distally and because the conical sections 55 and 57 of the wires 49 and 51 are of progressively decreasing diameter. The distal section 41 is more flexible than the transition section 39 because the coil turns 47 are contiguous and because wire of minimum diameter is used to construct it. If the wire 49 is used to construct the inner conductor 17, then the progressive decrease in diameter of the conical section 55 tends to produce progressively greater flexibility in the distal section 41 as the distal section extends distally. The distal section 41 is flexible and resilient all the way to the distal end 48.

In the embodiment of Figs. 1-3, a non-conductive elastomer 61 fills the central space within the coils 47, 45 and 33. The elastomer 61 helps insulate the conductors 17 and 25 from each other. In the embodiment illustrated, the elastomer 61 terminates, along with the distal section 41, at the distal end 48. In this embodiment, the presence of the elastomer 61 does not alter the above-described relative stiffness relationships of the sections 37, 39 and 41. The elastomer 61 fills the spaces between the coil 45 and the coil 33, and in addition, encases the coil 45 of the transition section 39 so that only the coil 47 is exposed to define an electrode 62. In this embodiment, the distal section 41 forms the electrode 62. If the elastomer 61 is eliminated, then it is preferred to encase the coil 45 of the transition section 39 in a suitable insulation jacket.

Fig. 5 shows how the catheter can be used by inserting it through the lumen of a guiding catheter 63. Except for a stiffening element 64, the guiding catheter 63 may be of conventional construction and may be a Swan-Ganz thermodilution catheter which is available from American Edwards Laboratories of Irvine, California. The guiding catheter 63, which may have multiple lumens extending longitudinally through a catheter body, may be inserted into the heart through a vein using conventional techniques, and following such insertion, a balloon 65 adjacent the distal end of the guiding catheter is lodged in the pulmonary artery 67. As shown in Fig. 5, the catheter 63 extends through the superior vena cava 66 and is formed into a curve 68 of about 180 degrees as it extends through the right atrium 69 and the right ventricle 71. The guiding catheter 63 has a port 72 leading from one of its lumens into the right ventricle 71.

In the embodiment illustrated, the stiffening element 64 is in the form of an elongated, flexible, resilient wire of metal or plastic bonded into the guiding catheter 63 outside of the lumen with which the port 72 communicates. In the preferred construction illustrated, the stiffening element 64 extends from a location in the right atrium 69 proximally of the port 72 continuously to a location in the right ventricle 71 located distally of the port 72. Thus, regions of the guiding catheter on the opposite sides of the ports 72 are stiffened, and such stiffening is controlled to cause the catheter 63 to form the relatively gentle curve 68 in the right heart without kinking as the catheter extends through the right heart to the pulmonary artery 67.

With the guiding catheter 63 positioned in the right heart as shown in Fig. 5, the catheter 11 can be inserted through a lumen of the guiding catheter 63 and out the port 72. As the catheter 11 continues its advancing movement, the electrode 62 contacts a wall 73 of the right ventricle and bends over or deflects along the wall due to the resilience of the distal section 41 all the way to the distal end 48. This causes the electrode 62 and the transition section 39 to resiliently flex and causes the electrode to lie against the wall 73 without penetrating the wall. A circuit can then be completed from the electrode 62 through the heart wall 73 and body fluids in the heart to the electrode 35. The flexibility of the catheter 11, and in particular, of the conductors 17 and 25 at, and distally of the distal electrode 35, maintains the distal electrode 62 in continuous engagement with the wall 73. Of course, the catheter 11 can be inserted directly through an artery or vein into the heart without using the guiding catheter 63. The catheter 11 is flexible throughout its length. However, the resilience and flexibility of the catheter are carefully controlled primarily at the sections 39 and 41 to provide insufficient column strength to penetrate the heart wall 73 and sufficient resilience to maintain contact between the distal electrode 62 and with the heart wall.

Fig. 3b shows a catheter 11a which is identical in all respects not shown or described herein to the catheter 11. Portions of the catheter 11a corresponding to portions of the catheter 11 are designated by corresponding reference numerals followed by the letter "a."

The only difference between the catheters 11 and 11a is that the inner conductor 17a of the catheter 11a is provided in two parts, i.e., a straight segment 75 and a coiled segment 77 appropriately joined together as by solder or welding. For this purpose, the distal end of the straight segment 75 is inserted within a few of the proximal coil turns of the coiled segment 77. The straight segment 75 defines the first section 37a and the coiled segment 77 defines the transition section 39a and the distal section 41a, i.e., the electrode 62a.

Fig. 4 shows a unipolar catheter 11c which is identical in all respects not shown or described herein to the bipolar catheter 11. The only difference between the catheters 11 and 11c is that the outer conductor 25 is replaced with a flexible tube 87 of a suitable biocompatible plastic material. Portions of the catheter 11c corresponding to portions of the catheter 11 are designated by corresponding reference numerals followed by the letter "c." As shown schematically in Fig. 4, with the unipolar catheter 11c, the circuit is completed through the heart wall 73 to ground, and to accomplish this, the patient is appropriately grounded.

## Claims

1. A flexible tip pacing catheter (11) for electrically stimulating the heart comprising :
an elongated body (15) having an elongated passage therein;
an elongated wire conductor (17) partially in said passage, said elongated wire conductor (17) having a distal end portion outside of said passage which is flexible and resilient along its length, said distal end portion terminating substantially at a distal end (48) of the catheter;
said flexible resilient distal end portion including a flexible electrode (62) outside said body and exposed at the outer periphery of the catheter (11) whereby current can be passed between said electrode (62) and the heart; and
said wire conductor (17) further having a first section (37) lying at least partially within said passage of said body (15) and being straight, said distal end portion of said wire conductor (17) having a distal section (41) extending to the distal end of the catheter and joined to said first section by a transition section (39) of said distal end portion, said distal end portion being wound into a coil (45, 47) to form said distal section (41), said transition section (39) and said flexible electrode (62), said distal section (41) being more flexible than said transition section (39) with said transition section (39) being more flexible than said first section (37), and said distal section (41) forming said electrode (62).

2. A catheter (11) according to claim 1, wherein said coil (45, 47) forms the whole of said distal end portion of said conductor.

3. A catheter according to claim 1 or 2, wherein the turns of the coil (45) of the transition section (39) are spaced by a greater axial distance than the turns of the coil (47) of the distal section (41).

4. A catheter (11) according to claim 2 or claim 3, wherein said wire conductor of said first section (37) has a greater cross-sectional area than the wire conductor of said transition section (39) and the wire conductor of said transition section (39) has a larger cross-sectional area than the wire conductor of said distal section (41).

5. A catheter according to claim 4, wherein the cross-sectional area of the wire conductor decreases step-wise.

6. A catheter (11) according to any one of claims 1 to 5, wherein the flexibility of the transition section (39) progressively decreases as the transition section (39) extends proximally.

7. A catheter (11) according to claim 6, wherein in said transition section (39) the wire conductor (17) is of progressively reducing cross-sectional area as such section extends distally.

8. A catheter according to any one of claims 1 to 7, wherein the catheter (11) also has a proximal electrode (35) spaced proximally from said transition section.

9. A catheter (11) according to claim 8, wherein said body (15) includes a conductive wire (25) having a plurality of coils (31, 33) with a region of said coils (31, 33) of said wire (25) being exposed to define the proximal electrode (35).

## Patentansprüche

1. Schrittmacherkatheter (11) mit flexibler Spitze zur elektrischen Stimulierung des Herzens mit:
einem einen langgestreckten Kanal enthaltenden, langgestreckten Körper (15),
einem teilweise im Kanal befindlichen, langgestreckten Drahtleiter (17), wobei der langgestreckte Drahtleiter (17) außerhalb des Kanals ein distales Endteil aufweist, das auf seiner Länge flexibel und federnd ist, wobei das distale Endteil im Wesentlichen an einem distalen Ende (48) des Katheters endet,
wobei das flexible, federnde, distale Endteil außerhalb des Körpers eine flexible Elektrode (62) aufweist, die am Außenumfang des Katheters (11) freiliegt, wodurch Strom zwischen der Elektrode (62) und dem Herzen geleitet werden kann,
wobei der Drahtleiter (17) ferner einen ersten Abschnitt (37) aufweist, der wenigstens teilweise im Kanal des Körpers (15) liegt und geradlinig ist, wobei das distale Endteil des Drahtleiters (17) einen distalen Abschnitt (41) besitzt, der sich zum distalen Ende des Katheters erstreckt und mit dem ersten Abschnitt durch einen Übergangsabschnitt (39) des distalen Endteils verbunden ist, wobei das distale Endteil zu einer Spirale (45, 47) gewickelt ist, um den distalen Endabschnitt (41), den Übergangsabschnitt (39) und die flexible Elektrode (62) zu bilden, wobei der distale Abschnitt (41) flexibler ist als der Übergangsabschnitt (39), wobei der Übergangsabschnitt (39) flexibler ist als der erste Abschnitt (37) und der distale Abschnitt (41) die Elektrode (62) bildet.

2. Katheter (11) nach Anspruch 1, bei dem die Spirale (45, 47) die Gesamtheit des distalen Endteils des Leiters bildet.

3. Katheter nach Anspruch 1 oder 2, bei dem die Windungen der Spirale (45) des Übergangsabschnittes (39) um eine größere axiale Strecke als die Windungen der Spirale (47) des distalen Abschnittes (41) beabstandet sind.

4. Katheter (11) nach Anspruch 2 oder 3, bei dem der Drahtleiter des ersten Abschnittes (37) eine größere Querschnittsfläche als der Drahtleiter des Übergangsabschnittes (39) und der Drahtleiter des Übergangsabschnittes (39) eine größere Querschnittsfläche als der Drahtleiter des distalen Abschnittes (41) besitzt.

5. Katheter nach Anspruch 4, bei dem die Querschnittsfläche des Drahtleiters schrittweise abnimmt.

6. Katheter (11) nach einem der Ansprüche 1 bis 5, bei dem die Flexibilität des Übergangsabschnittes (39) mit proximaler Erstreckung des Übergangsabschnitts (39) fortschreitend abnimmt.

7. Katheter (11) nach Anspruch 6, bei dem der Übergangsabschnitt (39) des Drahtleiters (17) mit distaler Erstreckung dieses Abschnitts eine fortschreitend abnehmende Querschnittsfläche besitzt.

8. Katheter nach einem der Ansprüche 1 bis 7, wobei der Katheter (11) ferner eine proximale Elektrode (35) aufweist, die vom Übergangsabschnitt proximal beabstandet ist.

9. Katheter (11) nach Anspruch 8, bei dem der Körper (15) einen leitenden Draht (25) mit einer Vielzahl von Spiralen (31, 33) enthält, wobei ein Bereich der Spiralen (31, 33) des Drahtes (25) zur Bildung der proximalen Elektrode (35) freiliegt.

## Revendications

1. Cathéter de stimulation cardiaque à extrémité flexible (11) pour stimuler électriquement le coeur, comprenant :
un corps allongé (15) ayant un passage allongé ménagé à l'intérieur ;
un fil conducteur allongé (17) situé partiellement dans ledit passage, ledit fil conducteur allongé (17) ayant une partie d'extremité distale qui se situe à l'extérieur dudit passage, cette partie étant flexible et élastique le long de sa longueur, ladite partie d'extrémité distale se terminant sensiblement au niveau d'une extrémité distale (48) du cathéter ;
ladite partie d'extrémité distale élastique et flexible incluant une électrode flexible (62) située à l'extérieur dudit corps et exposée au niveau de la périphérie externe du cathéter (11) et ainsi, un courant peut passer entre ladite électrode (62) et le coeur ; et
ledit fil conducteur (17) ayant en outre une première section (37) qui est située au moins partiellement à l'intérieur dudit passage dudit corps (15) et qui est rectiligne, ladite partie d'extrémité distale dudit fil conducteur (17) ayant une section distale (41) s'étendant jusqu'à proximité de l'extrémité distale du cathéter et qui est reliée à ladite première section par une section de transition (39) de ladite partie d'extrémité distale, ladite partie d'extrémité distale étant enroulée sous la forme d'une bobine (45, 47) de manière à former ladite section distale (41), ladite section de transition (39) et ladite électrode flexible (62), ladite section distale (41) étant plus flexible que ladite section de transition (39) et ladite section de transition (39) étant plus flexible que ladite première section (37), et ladite section distale (41) formant ladite électrode (62).

2. Cathéter (11) selon la revendication 1, dans lequel ladite bobine (45, 47) forme la totalité de ladite partie d'extrémité distale dudit conducteur.

3. Cathéter selon la revendication 1 ou 2, dans lequel les tours de la bobine (45) de la section de transition (39) sont espacés d'une distance axiale supérieure à celle qui sépare les tours de la bobine (47) de la section distale (41).

4. Cathéter (11) selon la revendication 2 ou 3, dans lequel ledit fil conducteur de ladite première section (37) a une section transversale plus grande que celle du fil conducteur de ladite section de transition (39) et le fil conducteur de ladite section de transition (39) a une section transversale plus grande que celle du fil conducteur de ladite section distale (41).

5. Cathéter selon la revendication 4, dans lequel la section transversale du fil conducteur diminue pas à pas.

6. Cathéter (11) selon l'une quelconque des revendications 1 à 5, dans lequel la flexibilité de la section de transition (39) diminue progressivement lorsque la section de transition (39) s'étend de manière proximale.

7. Cathéter (11) selon la revendication 6, dans lequel, dans ladite section de transition (39), le fil conducteur (17) voit sa section transversale se réduire progressivement lorsque cette section s'étend de manière distale.

8. Cathéter selon l'une quelconque des revendications 1 à 7, dans lequel le cathéter (11) comprend également une électrode proximale (35) espacée de manière proximale de ladite section de transition.

9. Cathéter (11) selon la revendication 8, dans lequel ledit corps (15) comprend un fil conducteur (25) qui comporte une pluralité de bobines (31, 33), une région desdites bobines (31, 33) dudit fil (25) étant exposée pour définir l'électrode proximale (35).
